# EUROPÄISCHE PATENTSCHRIFT

(11) **EP 1 251 853 B1**
(45) Veröffentlichungstag und Bekanntmachung des Hinweises auf die Patenterteilung: **29.10.2003**
(21) Anmeldenummer: 01902334.0
(22) Anmeldetag: 18.01.2001
(51) Int. Cl.: A61K 31/505, A61P 25/00

(54) **TRANSDERMALES THERAPEUTISCHES SYSTEM ZUR VERABREICHUNG VON ZALEPLON**
TRANSDERMAL THERAPEUTIC SYSTEM FOR THE ADMINISTRATION OF ZALEPLON
SYSTEME THERAPEUTIQUE TRANSDERMIQUE POUR L'ADMINISTRATION DE ZALEPLON

(30) Priorität: 01.02.2000 DE 10004790
(43) Veröffentlichungstag der Anmeldung: 30.10.2002
(73) Patentinhaber: LTS Lohmann Therapie-Systeme AG, 56626 Andernach (DE)
(72) Erfinder: SELZER, Thorsten, 56564 Neuwied (DE)
(74) Vertreter: Flaccus, Rolf-Dieter, Dr.
(86) Internationale Anmeldenummer: EP0100532
(87) Internationale Veröffentlichungsnummer: WO01056576

(56) Entgegenhaltungen:
- EP-A- 0 208 846
- DATABASE FILE REGISTRY (CA) [Online] American Chemical Society; RN: 151319-34-5, "Zaleplon" XP002164847

## Beschreibung

Die Erfindung betrifft eine Arzneimittelzubereitung mit dem Wirkstoff Zaleplon in Form eines transdermalen therapeutischen Systems. Die Erfindung umfaßt ferner die Verwendung von Zaleplon zur Herstellung von transdermalen therapeutischen Systemen zur Behandlung oder Prophylaxe von Krankheitszuständen oder gesundheitlichen Störungen, sowie ein Verfahren zur Herstellung solcher transdermaler therapeutischer Systeme.

Transdermale therapeutische Systeme (TTS) sind Darreichungsformen, die auf die Haut appliziert werden und dafür konzipiert sind, einen Arzneistoff nach transdermaler Aufnahme systemisch verfügbar zu machen. TTS können den therapeutischen Wert einer Arzneistoffverabreichung erhöhen, indem sie eine konstante Abgabe des Wirkstoffes über einen verlängerten Zeitraum in das Blutkompartiment gewährleisten. Auf diese Weise können auch Probleme wie gastrointestinale Intoleranz, niedrige enterale Absorption, "First-Pass"-Metabolisierung in der Leber und erhöhte Applikationsfrequenz bei niedrigen Halbwertszeiten umgangen werden.

TTS bestehen nach dem Stand der Technik üblicherweise aus einer arzneistoffundurchlässigen Trägerschicht, einer arzneistoffhaltigen Reservoirschicht sowie einer Haftklebeschicht zur Befestigung auf der Haut, wobei diese mit der arzneistoffhaltigen Reservoirschicht identisch sein kann, und einer vor der Applikation zu entfernenden arzneistoffundurchlässigen Schutzschicht. Zusätzlich kann eine Steuermembran vorhanden sein. Die arzneistoffhaltige Reservoirschicht besteht aus Arzneistoff (wirkstoff) und Hilfsstoffen, wie z. B. Weichmacher, Klebrigmacher, Lösungsvermittler, Stabilisatoren, Füllstoffe, Trägerstoffe und Permeationsbeschleuniger.

Der Wirkstoff Zaleplon, ein Pyrazolopyrimidin, stellt ein neuartiges Schlafmittel aus der Klasse der Non-Benzodiazepine dar. Die Effekte von Zaleplon werden über agonistische Wirkung am Benzodiazepin-Omegal-(Typl)-Rezeptorsubtyp des GABA(A)-Rezeptorkomplexes erklärt (Scharf M., A new Option for insomnia., Health News, 1999 Oct. 1, 5(12):4; Annseau M., Pharma-clinics, Drug of the month. Zaleplon. Rev. Med Liege 1999, Aug; 54(8):705-6).

Schlaf ist ein ständig wiederkehrender Erholungsvorgang des Körpers. Er verläuft in unterschiedlichen Phasen:
a) der Tiefschlaf mit verlangsamten hirnelektrischen Wellen; dieser wird auch als passiver Erholungsschlaf bezeichnet. Hier laufen in vielen Organen Regenerations- und Aufbauvorgänge ab.
b) der Traumschlaf mit flachen hirnelektrischen Wellen und raschen Augenbewegungen; er dient vor allem der Weiterverarbeitung von tagsüber aufgenommenen Informationen.

Beim normalen Schlaf lösen sich nach dem Einschlafstadium die beiden Schlafphasen vier- bis fünfmal ab. Beide Schlafarten sind zur körperlichen und psychischen Erholung absolut notwendig. Schlafstörungen liegen dann vor, wenn dieser Erholungsvorgang beträchtlich gestört ist - sei es durch eine Änderung der Schlafdauer oder des Ablaufs der Schlafphasen.
Etwa 10 % der Bevölkerung leiden unter chronischen und ca. 50 % unter gelegentlich vorkommenden Schlafstörungen.

Zaleplon wurde im Rahmen einer klinischen Studie bei über 3700 Patienten in USA, Kanada und Europa untersucht, auch bei älteren Menschen im Alter zwischen 65 und 85 Jahren. Es erwies sich bei Patienten mit Einschlafstörungen als sehr wirksam. Im Vergleich zu Placebo reduzierte es in klinischen Studien signifikant sowohl die polysomnographisch als auch subjektiv eingeschätzte Schlaflatenz. Die Schlafstadien 3 und 4 (Delta- bzw. Tiefschlaf) werden durch Zaleplon nicht verschlechtert. Die Auswirkungen auf den REM-Schlaf, die Gesamtschlafdauer und die Schlafeffizienz waren in den einzelnen Studien uneinheitlich und sind möglicherweise dosisabhängig (12^{th} Annual Meeting of the Assoc. Prof. Sleep Soc. 7/15/98, New Orleans).

Allerdings weist Zaleplon auch gewisse Nachteile auf, die sich aus der Pharmakokinetik ergeben: Zaleplon wird nach oraler Gabe rasch resorbiert, die absolute Verfügbarkeit baträgt aber lediglich 30 %. Die Plasmahalbwertszeit liegt bei nur einer Stunde. Dies kann dazu führen, daß die orale Gabe von Zaleplon zwar die Schlaflatenz verkürzt und den Schlafeintritt herbeiführt, daß aber ein Durchschlafen nicht immer gewährleistet wird, so daß die Patienten zwischenzeitlich erwachen und gegebenenfalls eine weitere Dosis zu sich nehmen müssen. Zu beachten ist, daß schlaffördernde Mittel grundsätzlich in möglichst niedriger Dosis verabreicht werden sollten. Deshalb ist eine Erhöhung der oralen Dosis, um insbesondere das Durchschlafen zu fördern, nur sehr begrenzt möglich.

Die der vorliegenden Erfindung zugrundeliegende Aufgabe bestand deshalb darin, eine Zaloplon enthaltende Arzneizubereitung bereitzustellen, welche die mit der oralen Verabreichung des Wirkstoffs Zaleplon auftretenden, oben beschriebenen Nachteile weitestgehend vermeidet. Zugleich soll die Arzneizubereitung für die Behandlung von Einschlaf- bzw. Durchschlafstörungen geeignet sein.

Diese Aufgabe wird erfindungsgemäß durch eine Arzneizubereitung nach Anspruch 1 gelöst, gemäß welchem die Zaleplon enthaltende Arzneizubereitung ein transdermales therapeutisches System (TTS) in Pflasterform darstellt, wobei der Wirkstoff Zaleplon im Wirkstoffreservoir des TTS enthalten ist. Das erfindungsgemäße TTS kann verwendet werden, um auf dem Wege der transdermalen Verabreichung von Zaleplon Einschlaf- und / oder Durchschlafstörungen zu behandeln.

Eine Voraussetzung für die transdermale Verabreichung von Zaleplon zur Behandlung von Schlafstörungen ist, daß ein hoher Wirkstoffflux in vivo erreicht wird, was bei den erfindungsgemäßen transdermalen Zubereitungen der Fall ist. Bei Verwendung der erfindungsgemäßen Zaleplon enthaltenden TTS durch Applikation auf die Haut ergibt sich, anders als bei der oralen Verabreichung, eine weitgehend konstante Abgabe des Wirkstoffes im Verlauf des Applikationszeitraumes. Auf diese Weise wird die an sich rasch stattfindende Elimination des Wirkstoffes Zaleplon durch ständiges Nachliefern aus dem Wirkstoffreservoir des TTS kompensiert bzw. ausgeglichen. Zudem wird auf diese Weise das Problem der oralen Verfügbarkeit umgangen.
Die transdermale Verabreichung des schlaffördernden Mittels kann insbesondere bei solchen Patienten von Vorteil sein, die aufgrund anderer bestehender Krankheiten bereits eine größere Anzahl verschiedene Medikamente oral einnehmen müssen. Ferner ist die transdermale Verabreichung für den Patienten auch sicherer und kontrollierbarer, z. B. bei auftretenden Gedächtnisstörungen, welche unter Umständen dazu führen können, daß ein oral zu verabreichendes Mittel versehentlich mehrmals in kurzer Folge eingenommen wird, wodurch es zu einer Überdosierung kommen könnte.

Die Applikation der erfindungsgemäßen pharmazeutischen Zubereitungen kann derartig erfolgen, daß entsprechende TTS in Form von Pflastern ca. 2 h vor dem Schlafengehen auf die Haut geklebt werden und nach dem morgendlichen Aufstehen wieder entfernt werden. Aufgrund der raschen Elimination und kurzen Halbwertszeit klingt die Wirkung nach Entfernen des Pflasters rasch ab, so daß sich keine "hangover"-Effekte ergeben, wie dies bei vielen anderen Durchschlafmitteln der Fall ist.

Grundsätzlich können die erfindungsgemäßen TTS sowohl in Form von Matrixsystemen, als auch in Form von Membran- bzw. Beutelreservoirsystemen eingesetzt werden. Bezüglich der Verwendung von Polymeren, Harzen und weiteren Zusatzstoffen bestehen keine Einschränkungen, außer, daß die Formulierung kompatibel mit dem Wirkstoff Zaleplon sein muß und daß sie geeignet sein muß, diesen Wirkstoff an die Haut abzugeben. Im einfachsten Fall kann ein erfindungsgemäßes TTS erhalten werden, indem Zaleplon in einer Lösung von Grundpolymeren grob (d. h. partikulär), kolloidal oder molekular dispergiert bzw. gelöst wird, und diese Mischung auf eine geeignete Unterlage - in der Regel eine silikonisierte thermoplastische Folie (die spätere Schutzschicht) - beschichtet wird. Nach Trocknen bzw. Abdampfen der Lösemittelanteile wird die erhaltene Schicht, welche das haftklebende Wirkstoffreservoir darstellt, mit einer weiteren Folie abgedeckt, welche die spätere Rückschicht des TTS darstellt. Aus diesem Laminat können anschließend durch Stanzen flächiger Gebilde TTS in der gewünschten geometrischen Form erhalten werden.

Der Aufbau der erfindungsgemäßen TTS umfaßt neben einer Wirkstoffmatrix, die auch als beutelförmiges Reservoir gestaltet sein kann, eine wirkstoffundurchlässige Rückschicht sowie eine ebenfalls wirkstoffundurchlässige, abziehbare Schutzfolie.
Als Rückschicht eignen sich vor allem Polyester, welche sich durch besondere Festigkeit auszeichnen, darüber hinaus aber auch nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid oder ähnlicher Stoffe, die dem Fachmann bekannt sind.
Für die ablösbare Schutzfolie können dieselben Materialien verwendet werden wie für die Rückschicht, vorausgesetzt, daß sie durch geeignete Oberflächenbehandlung, wie z. B. silikonisierung, ablösbar ist. Es können aber auch andere ablösbare Schutzschichten, wie Polytetrafluorethylenbehandeltes Papier, Cellophan, Polyvinylchlorid, oder ähnliche verwendet werden.

Geeignete Grundstoffe für die erfindungsgemäße Zubereitung in Form eines TTS, insbesondere für die Herstellung der Matrixschicht(en), sind vor allem Polymere auf Basis von Acrylsäure und deren Ester, Polyacrylaten, Isobutylen, Ethylen-Vinylacetat, Kautschuken, Styrol-Dien-Copolymeren, Synthesekautschuken oder Heißschmelzklebern. Diese Aufzählung ist bei weitem nicht vollständig, läßt aber die breite Anwendungsfähigkeit des erfindungsgemäßen Prinzips erkennen.

Das erfindungsgemäße TTS kann weiterhin dadurch gekennzeichnet sein, daß der Wirkstoff vorzugsweise in gelöstem Zustand vorliegt, wobei die Formulierung möglichst einen Lösungsvermittler enthalten sollte. Hierfür können vor allem mehrwertige Alkohole, besonders bevorzugt 1,2-Propandiol, verwendet werden. Weitere Beispiele für geeignete Lösungsvermittler sind Tetrahydrofurfurylalkohol, Transcutol, Butandiol, Glycerin, PEG 400, Diethyltoluamid, Monoisopropylidenglycerin.
Der Anteil des Lösungsvermittlers, bezogen auf die Masse des fertigen TTS, kann zwischen 1 % und 50 % betragen, wobei vorzugsweise ein Massen-Anteil zwischen 5 % und 35 % gewählt wird.

Um einen hohen Wirkstoffflux zu erreichen, hat es sich ferner als besonders vorteilhaft erwiesen, dem Wirkstoffreservoir (und gegebenenfalls auch weiteren Schichten des TTS) einen Hautpenetrationsverstärker ("Enhancer") hinzuzufügen. Dessen Konzentration liegt vorzugsweise im Bereich von 0,1 % bis 25 %, besonders bevorzugt im Bereich von 1 % und 10 %, jeweils bezogen auf die Masse des TTS.
Beispiele für geeignete Enhancer sind Decanol, Dodecanol, Ölsäure, Ölsäurediethanolamin, Myristinsäure, Sorbitanmonolaurat, Polyoxylaurylether. Vorzugsweise wird Polyoxylaurylether Brij® eingesetzt.
Des weiteren können die erfindungsgemäßen Zaleplon enthaltenden TTS auch zwei oder mehrere Enhancerverbindungen in Kombination enthalten.

Um eine hohe Freisetzungsrate zu erzielen, wird vorzugsweise eine möglichst hohe Wirkstoffkonzentration in den wirkstoffhaltigen Schichten angestrebt. Dabei ist allerdings zu beachten, daß bei zu hohen Konzentrationen die physikalische Stabilität beeinträchtigt werden kann.
Bei den erfindungsgemäßen TTS werden deshalb Wirkstoffkonzentrationen im Bereich von 0,1 % bis 50 %, bevorzugt im Bereich von 1 % bis 10 % verwendet, jeweils bezogen auf die Gesamtmasse der wirkstoffhaltigen Schichten.

Gemäß einer besonderen Ausführungsform können die erfindungsgemäßen TTS auch zwei oder mehrere wirkstoffhaltige Matrixschichten aufweisen. Dabei können die einzelnen Matrixschichten unterschiedliche Konzentrationen an Wirkstoff, Enhancer oder Lösungsvermittler aufweisen. Ferner können die einzelnen Matrixschichten sich auch durch unterschiedliche Haftkleber auszeichnen.
Sofern die Steuerung der Wirkstofffreisetzung nicht durch andere Mechanismen bewirkt wird, kann das Reservoir auch mit einer Steuermembran versehen werden, welche die Abgabe des Wirkstoffes an die Haut steuert.

Gemäß einer anderen bevorzugten Ausführungsform kann der Wirkstoff auch in einem beutelförmigen Reservoir vorliegen, welches mit einer flüssigen, hochviskosen, halbfesten oder thixotropen Matrix gefüllt ist. Dabei ist es besonders vorteilhaft, wenn das halbfeste oder thixotrope Wirkstoffreservoir einen Gelbildner enthält. Die der Haut abgewandte Beutelrückseite muß dabei Wirkstoff undurchlässig, die der Haut zugewandte Seite Wirkstoff durchlässig sein. Optional kann eine wirkstoffdurchlässige Membran (Steuermembran) die Steuerung der Wirkstofffreisetzung übernehmen.

Bei der Herstellung der erfindungsgemäßen TTS kann beispielsweise nach folgender Methode vorgegangen werden: Zunächst wird der Wirkstoff Zaleplon sowie ein geeigneter Enhancer, z. B. Brij®30, in einem Lösungsvermittler, z. B. 1,2-Propandiol, gelöst, wobei die Konzentration von Zaleplon möglichst die Sättigungslöslichkeit erreichen sollte. Gegebenenfalls kann die Lösung auch übersättigt sein. Diese Lösung wird mittels einer geeigneten Rührapparatur in den Silikonkleber, der ebenfalls in einem Lösungsmittel gelöst ist, gegeben und dispergiert, so daß eine möglichst homogene Flüssig-Flüssig-Dispersion entsteht. Diese Dispersion wird mit einer geeigneten Vorrichtung gleichmäßig auf eine Trägerfolie beschichtet. Anschließend werden durch eine kontrollierte Trocknung das Lösungsmittel des Silikonklebers sowie eventuelle Anteile des Lösungsvermittlers entfernt. Das so erhaltene Laminat wird danach mit einer weiteren Folie zukaschiert. Zuletzt werden TTS einer bestimmten Fläche ausgestanzt und in ein adäquates Packmittel verpackt.

Das primäre Anwendungsgebiet der erfindungsgemäßen Zaleplon enthaltenden TTS ist die Behandlung von Schlafstörungen, d. h. von Einschlaf- und Durchschlafstörungen.
Nach der Erfindung können Zaleplon enthaltende TTS auch bei weiteren Indikationen eingesetzt werden. Beispielsweise eignen sich die erfindungsgemäßen Zaleplon-enthaltenden TTS für die Behandlung akuter und chronischer Spannungs-, Erregungs- oder Angstzustände, sowie für die Behandlung von Zuständen mit erhöhtem Muskeltonus, z. B. zur Behandlung oder Prophylaxe von Muskelspasmen oder Muskelverspannungen. Ferner können die Zaleplon enthaltende TTS auch als Prämedikation vor chirurgischen oder diagnostischen Eingriffen, zur Unterstützung einer Narkose, sowie als postoperative Medikation eingesetzt werden.
Die Zaleplon enthaltenden TTS können auch zur Prophylaxe oder Behandlung bestimmter psychischer Störungen eingesetzt werden, z. B. bei Psychosen aus dem schizophrenen Formenkreis, bei Angstzuständen oder bei Depressionen. Ebenso ist erfindungsgemäß die Anwendung Zaleplon-enthaltender TTS zur Behandlung oder Prophylaxe von Migräne oder von epileptischen Anfällen vorgesehen.

## Patentansprüche

1. Zaleplon enthaltende pharmazeutische Zubereitung, **dadurch gekennzeichnet, daß** die Zubereitung ein transdermales therapeutisches System in Pflasterform darstellt, mit einer Rückschicht, einem damit verbundenen haftklebenden Wirkstoffreservoir und einer vor der Applikation ablösbaren Schutzschicht, wobei das wirkstoffreservoir Zaleplon enthält.

2. Zubereitung nach Anspruch 1, **dadurch gekennzeichnet, daß** sie mindestens einen Lösungsvermittler enthält, vorzugsweise aus der Gruppe der mehrwertigen Alkohole, besonders bevorzugt 1,2-Propandiol.

3. Zubereitung nach Anspruch 1 oder 2, **dadurch gekennzeichnet, daß** sie mindestens einen Haut-Penetrationsverstärker enthält, vorzugsweise aus der Gruppe, die Polyoxyethylenfettalkoholether, besonders bevorzugt Brij®30, sowie Polyoxyethylenfettsäureester, Polyoxyethylensorbitanfettsäureester, Sorbitanfettsäureester, Fettsäuren, Fettalkohole, Ester von Fettsäuren mit Methanol oder Ethanol oder Isopropanol, Ester von Fettalkoholen mit Essigsäure oder Milchsäure umfaßt.

4. Zubereitung nach Anspruch 3, **dadurch gekennzeichnet, daß** sie eine Kombination von mindestens zwei Penetrationsverstärkern enthält.

5. Zubereitung nach einem der Ansprüche 1 bis 4, **dadurch gekennzeichnet, daß** für das haftklebende Wirkstoffreservoir ein Haftkleber verwendet wird, der ausgewählt ist aus der Gruppe, die Silikonhaftkleber, Haftkleber auf der Basis von Polyacrylaten, Polyisobutylenen, Polyterpenen, Ethylenvinylacetat-Copolymeren, Kautschuken, Synthesekautschuken oder Heißschmelzklebern umfaßt, wobei vorzugsweise Kombinationen der genannten Haftschmelzkleber verwendet werden.

6. Zubereitung nach einem oder mehreren der vorangehenden Ansprüche, **dadurch gekennzeichnet, daß** das transdermale therapeutische System einen schichtförmigen Aufbau mit mindestens zwei Polymermatrixschichten besitzt, wobei vorzugsweise mindestens eine der Matrixschichten Polymerbestandteile enthält, die aus den in Anspruch 5 genannten Stoffen ausgewählt sind.

7. Zubereitung nach einem oder mehreren der vorangehenden Ansprüche, **dadurch gekennzeichnet, daß** mindestens eine Matrixschicht des transdermalen therapeutischen Systems Polymerbestandteile enthält, die aus der Gruppe der substituierten Cellulosen ausgewählt sind, vorzugsweise der Methyloder Ethylcellulosen.

8. Zubereitung nach einem oder mehreren der vorangehenden Ansprüche, **dadurch gekennzeichnet, daß** sie Weichmacher in einer Konzentration von 0 bis 30 Gew.-%, vorzugsweise von 5-20 Gew.-% enthält, wobei die Weichmacher ausgewählt sind aus der Gruppe, die Kohlenwasserstoffe, Alkohole, Carbonsäuren und ihre Derivate, Ether, Ester und Amine umfaßt.

9. Zubereitung nach einem oder mehreren der vorangehenden Ansprüche, **dadurch gekennzeichnet, daß** das Wirkstoffreservoir als beutelförmiges Reservoir gestaltet ist, welches mit einer flüssigen, hochviskosen, halbfesten oder thixotropen Matrix gefüllt ist, die den Wirkstoff Zaleplon enthält.

10. Verwendung von Zaleplon zur Herstellung eines transdermalen therapeutischen Systems zur Behandlung von Einschlafoder Durchschlafstörungen durch transdermale Verabreichung des Wirkstoffs Zaleplon.

11. Verwendung von Zaleplon zur Herstellung eines transdermalen therapeutischen Systems zur Behandlung akuter und chronischer Spannungs-, Erregungs- oder Angstzustände.

12. Verwendung von Zaleplon zur Herstellung eines transdermalen therapeutischen Systems zur Behandlung von Zuständen mit erhöhtem Muskeltonus.

13. Verwendung von Zaleplon nach Anspruch 12, **dadurch gekennzeichnet, daß** das genannte System für die Therapie oder Prophylaxe von Muskelspasmen oder Muskelverspannungen bestimmt ist.

14. Verwendung nach einem der Ansprüche 10 bis 13, **dadurch gekennzeichnet, daß** das genannte System für die Prämedikation vor chirurgischen oder diagnostischen Eingriffen, oder für die postoperative Medikation bestimmt ist.

15. Verwendung nach einem der Ansprüche 10 bis 13, **dadurch gekennzeichnet, daß** das genannte System für die Unterstützung einer Narkose bestimmt ist.

16. Verwendung von Zaleplon zur Herstellung eines transdermalen therapeutischen Systems zur Behandlung oder Prophylaxe von Psychosen aus dem schizophrenen Formenkreis.

17. Verwendung von Zaleplon zur Herstellung eines transdermalen therapeutischen Systems zur Behandlung oder Prophylaxe von Depressionen.

18. Verwendung von Zaleplon zur Herstellung eines transdermalen therapeutischen Systems zur Behandlung oder Prophylaxe von epileptischen Anfällen.

19. Verwendung von Zaleplon zur Herstellung eines transdermalen therapeutischen Systems zur Behandlung oder Prophylaxe von Migräne.

20. Verfahren zur Herstellung eines Zaleplon enthaltenden transdermalen therapeutischen Systems nach einem der Ansprüche 1 bis 9, **dadurch gekennzeichnet, daß** der Wirkstoff Zaleplon sowie ein Hautpenetrationsverstärker in einem Lösungsvermittler gelöst werden, wobei die Konzentration von Zaleplon möglichst die Sättigungslöslichkeit erreichen sollte, und daß diese Lösung anschließend unter Rühren in einer Haftkleberlösung dispergiert wird, die entstandene Dispersion auf eine Trägerfolie beschichtet wird, nach erfolgter Trocknung eine weitere Folie auflaminiert wird, und schließlich transdermale therapeutische Systeme mit einer bestimmten Fläche ausgestanzt und in ein Packmittel verpackt werden.

## Claims

1. A zaleplon-containing pharmaceutical preparation, **characterized in that** said preparation is a transdermal therapeutic system in plaster form, having a backing layer, having a pressure-sensitive adhesive active ingredient reservoir connected thereto, and having a protective layer which can be detached before application, where the active ingredient reservoir contains zaleplon.

2. The preparation as claimed in claim 1, **characterized in that** it contains at least one solubilizer, preferably from the group of polyhydric alcohols, particularly preferably 1,2-propanediol.

3. The preparation as claimed in claim 1 or 2, **characterized in that** it contains at least one skin penetration enhancer, preferably from the group comprising polyoxyethylene fatty alcohol ethers, particularly preferably Brij® 30, and polyoxyethylene fatty acid esters, polyoxyethylene sorbitan fatty acid esters, sorbitan fatty acid esters, fatty acids, fatty alcohols, esters of fatty acids with methanol or ethanol or isopropanol, esters of fatty alcohols with acetic acid or lactic acid.

4. The preparation as claimed in claim 3, **characterized in that** it contains a combination of at least two penetration enhancers.

5. The preparation as claimed in any of claims 1 to 4, **characterized in that** the pressure-sensitive adhesive used for the pressure-sensitive adhesive active ingredient reservoir is selected from the group comprising silicone pressure-sensitive adhesives, pressure-sensitive adhesives based on polyacrylates, polyisobutylenes, polyterpenes, ethylene/vinyl acetate copolymers, rubbers, synthetic rubbers or hot melt adhesives, with use of combinations of said hot melt pressure-sensitive adhesives being preferred.

6. The preparation as claimed in one or more of the preceding claims, **characterized in that** the transdermal therapeutic system has a layered structure with at least two polymer matrix layers, with at least one of the matrix layers preferably comprising polymer constituents selected from the materials mentioned in claim 5.

7. The preparation as claimed in one or more of the preceding claims, **characterized in that** at least one matrix layer of the transdermal therapeutic system comprises polymer constituents selected from the group of substituted celluloses, preferably of methyl- or ethylcelluloses.

8. The preparation as claimed in one or more of the preceding claims, **characterized in that** it contains plasticizers in a concentration of from 0 to 30% by weight, preferably of 5-20% by weight, where the plasticizers are selected from the group comprising hydrocarbons, alcohols, carboxylic acids and derivatives thereof, ethers, esters and amines.

9. The preparation as claimed in one or more of the preceding claims, **characterized in that** the active ingredient reservoir is designed as reservoir which is in pouch form and which is filled with a liquid, highly viscous, semisolid or thixotropic matrix which comprises the active ingredient zaleplon.

10. The use of zaleplon for producing a transdermal therapeutic system for the treatment of difficulties in falling asleep or sleeping through, by transdermal administration of the active ingredient zaleplon.

11. The use of zaleplon for producing a transdermal therapeutic system for the treatment of acute and chronic states of tension, agitation or anxiety.

12. The use of zaleplon for producing a transdermal therapeutic system for the treatment of conditions with increased muscle tone.

13. The use of zaleplon according to claim 12, **characterized in that** the said system is for the purpose of therapy or prophylaxis of muscle spasms or muscle tenseness.

14. The use as claimed in any one of claims 10 to 13, **characterized in that** the said system is for the purpose of premedication before surgical or diagnostic interventions, or for postoperative medication.

15. The use as claimed in any one of claims 10 to 13, **characterized in that** the said system is for the purpose of assisting anesthesia.

16. The use of zaleplon for producing a transdermal therapeutic system for the treatment or prophylaxis of psychoses of the schizophrenic type.

17. The use of zaleplon for producing a transdermal therapeutic system for the treatment or prophylaxis of depressions.

18. The use of zaleplon for producing a transdermal therapeutic system for the treatment or prophylaxis of epileptic seizures.

19. The use of zaleplon for producing a transdermal therapeutic system for the treatment or prophylaxis of migraine.

20. A process for producing a zaleplon-containing transdermal therapeutic system as claimed in any one of claims 1 to 9, **characterized by** dissolving the active ingredient zaleplon and a skin penetration enhancer in a solubilizer, where the concentration of zaleplon should if possible reach the saturation solubility, and subsequently dispersing this solution by stirring in a pressure-sensitive adhesive solution, coating the resulting dispersion onto a support sheet and, after drying has taken place, laminating on another sheet, and finally punching out transdermal therapeutic systems with a defined area and packing them in packaging.

## Revendications

1. Préparation pharmaceutique contenant du zaleplon, **caractérisée en ce que** la préparation représente un système thérapeutique transdermique sous forme de pansement, avec une couche postérieure, un réservoir de substance active autoadhésive liée à celle-ci, et une couche protectrice détachable avant l'application, le réservoir de substance active contenant du zaleplon.

2. Préparation selon la revendication 1, **caractérisée en ce qu'**elle contient au moins un agent de solubilisation, de préférence issu du groupe des alcools plurivalents, de manière particulièrement préférée le 1,2-propanediol.

3. Préparation selon la revendication 1 ou 2, **caractérisée en ce qu'**elle contient au moins un agent renforçant la pénétration cutanée, de préférence issu du groupe qui comprend les éthers d'alcools gras polyoxyéthyléniques, de manière particulièrement préférée Brij® 30, ainsi que les esters d'acides gras polyoxyéthyléniques, les esters d'acides gras de polyoxyéthylène-sorbitan, les esters d'acides gras et de sorbitan, les acides gras, les alcools gras, les esters d'acides gras avec le méthanol ou l'éthanol ou l'isopropanol, les esters d'alcools gras avec l'acide acétique ou l'acide lactique.

4. Préparation selon la revendication 3, **caractérisée en ce qu'**elle contient une combinaison d'au moins deux agents renforçant la pénétration.

5. Préparation selon l'une quelconque des revendications 1 à 4, **caractérisée en ce qu'**une colle autoadhésive est employée pour le réservoir autoadhésif de substance active, qui est choisie dans le groupe qui comprend les colles autoadhésives à base de silicone, les colles autoadhésives à base de polyacrylates, de polyisobutylènes, de polyterpènes, de copolymères éthylène/acétate de vinyle, de caoutchoucs, de caoutchoucs synthétiques, ou de colles autoadhésives, des combinaisons des colles autoadhésives citées étant employées de préférence.

6. Préparation selon l'une ou plusieurs des revendications précédentes, **caractérisée en ce que** le système thérapeutique transdermique possède une structure stratiforme avec au moins deux couches de matrice polymère, de préférence au moins une des couches de matrice contenant des composants polymères qui sont choisis parmi les substances citées selon la revendication 5.

7. Préparation selon l'une ou plusieurs des revendications précédentes, **caractérisée en ce qu'**au moins une couche de matrice du système thérapeutique transdermique contient des composants polymères, qui sont choisis dans le groupe des celluloses substituées, de préférence les méthyl- ou éthyl-celluloses.

8. Préparation selon l'une ou plusieurs des revendications précédentes, **caractérisée en ce qu'**elle contient des plastifiants en une concentration allant de 0 à 30% en poids, de préférence de 5 à 20% en poids, les plastifiants étant choisis dans le groupe qui comprend les hydrocarbures, les alcools, les acides carboxyliques et leurs dérivés, les éthers, les esters et les amines.

9. Préparation selon l'une ou plusieurs des revendications précédentes, **caractérisée en ce que** le réservoir de substance active est réalisé sous la forme d'un réservoir en forme de sachet, lequel est rempli d'une matrice liquide, très visqueuse, semi-solide, ou thixotropique, qui contient la substance active, le zaleplon.

10. Utilisation du zaleplon pour la préparation d'un système thérapeutique transdermique pour traiter les troubles de l'endormissement ou du sommeil, au moyen de l'administration transdermique de la substance active, le zaleplon.

11. Utilisation du zaleplon pour préparer un système thérapeutique transdermique pour traiter les états de stress, d'excitation ou d'anxiété, aigus et chroniques.

12. Utilisation du zaleplon pour préparer un système thérapeutique transdermique pour traiter les états ayant un tonus musculaire surélevé.

13. Utilisation du zaleplon selon la revendication 12, **caractérisée en ce que** le système cité est destiné à la thérapie ou à la prophylaxie de spasmes musculaires ou de contractures musculaires.

14. Utilisation selon l'une quelconque des revendications 10 à 13, **caractérisée en ce que** le système cité est destiné à la prémédication avant des interventions chirurgicales ou de diagnostic, ou à la médication post-opératoire.

15. Utilisation selon l'une quelconque des revendications 10 à 13, **caractérisée en ce que** le système cité est destiné à assister une narcose.

16. Utilisation du zaleplon pour préparer un système thérapeutique transdermique destiné au traitement ou à la prophylaxie de psychoses issues des formes d'expression de la schizophrénie.

17. Utilisation du zaleplon pour préparer un système thérapeutique transdermique destiné au traitement ou à la prophylaxie de dépressions.

18. Utilisation-duzaleplon pour préparer un système thérapeutique transdermique destiné au traitement ou à la prophylaxie de crises d'épilepsie.

19. Utilisation du zaleplon pour préparer un système thérapeutique transdermique destiné au traitement ou à la prophylaxie de migraines.

20. Procédé de préparation d'un système thérapeutique transdermique contenant du zaleplon selon l'une quelconque des revendications 1 à 9, **caractérisé en ce que** la substance active, le zaleplon, ainsi qu'un agent renforçant la pénétration cutanée, sont dissous dans un agent de solubilisation, la concentration du zaleplon devant atteindre si possible la solubilité de saturation, et **en ce que** cette solution est dispersée ensuite sous agitation dans une solution de colle autoadhésive, la dispersion formée est déposée sur une feuille support, et stratifiée après séchage réussi d'une feuille supplémentaire et finalement des systèmes thérapeutiques transdermiques ayant une surface déterminée sont découpés à l'emporte-pièce et conditionnés dans un matériau d'emballage.
